(19) **Europäisches Patentamt**
**European Patent Office**
**Office européen des brevets**

(11) **EP 3 081 164 B1**

(12) **EUROPEAN PATENT SPECIFICATION**

(45) Date of publication and mention
of the grant of the patent:
**29.04.2020 Bulletin 2020/18**

(51) Int Cl.:
*A61B 5/153* (2006.01)   *A61B 5/15* (2006.01)
*A61B 5/154* (2006.01)   *A61M 25/06* (2006.01)
*A61M 5/158* (2006.01)

(21) Application number: **16170891.2**

(22) Date of filing: **07.03.2008**

(54) **FLASHBACK BLOOD COLLECTION NEEDLE**

FLASHBACK-BLUTENTNAHMENADEL

AIGUILLE DE PRÉLÈVEMENT SANGUIN À REFOULEMENT

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**19.10.2016 Bulletin 2016/42**

(62) Document number(s) of the earlier application(s) in accordance with Art. 76 EPC:
**08731700.4 / 2 254 472**

(73) Proprietor: **Becton, Dickinson and Company
Franklin Lakes, NJ 07417-1880 (US)**

(72) Inventors:
• **Tan, Chee Leong Alvin
510225 Singapore (SG)**

• **Moh, Jon
681683 Singapore (SG)**
• **Sim, Stanley
8100 Malaysia (MY)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(56) References cited:
**WO-A-2006/022716    US-A1- 2003 105 414
US-A1- 2006 036 219**

EP 3 081 164 B1

Printed by Jouve, 75001 PARIS (FR)

**Description**

BACKGROUND OF THE INVENTION

1. Field of the Invention

The present invention relates to a device for collecting blood samples by performing venipuncture on a patient. More particularly, the present invention relates to a needle assembly for multiple sample blood collection that allows a phlebotomist to determine whether vein entry has occurred when collecting a blood sample from a patient into an evacuated blood collection tube.

2. Description of Related Art

Venipuncture is the primary method used for acquiring blood samples for laboratory testing. In performing venipuncture procedures, a phlebotomist must follow several steps simultaneously. Such steps include assessing the patient's overall physical and psychological condition so as to properly select a venipuncture site and technique. The phlebotomist must also select the proper corresponding equipment, perform the technique so as to control bleeding, and properly collect and identify fluid specimens for testing. The phlebotomist must ascertain all of these coinciding factors, as such factors may adversely affect the distension of the vein and the length of the venipuncture procedure.

Various venipuncture devices have been developed to address the above-described problems. These devices include products intended to assist the phlebotomist in confirming that vein entry has been made see e.g. United States. Patent Nos. 5,222,502 and 5,303,713. Such a device contains a needle assembly with a housing that defines a chamber therein. A single cannula pointed at both ends is affixed to the housing. The intravenous (IV) end of the cannula is adapted for penetration of a patient's vein. The non-patient end of the cannula has a sealable sleeve and is adapted for penetration of a penetrable stop positioned within an evacuated container.

WO2006/022716 discloses a needle assembly comprising the features of the characterising portion of claim 1

Upon vein entry with the intravenous end of the cannula, blood will flow through the cannula, into the sealable sleeve and into the housing chamber, which is clear or translucent for visualization ("flashback"). Once air is vented from the flashback chamber, the blood therein is pressurized each time the sealable sleeve is pushed toward the housing chamber upon activation of an evacuated container.

Due to the length of time between vein entry and flashback, the phlebotomist may erroneously believe that satisfactory vein entry has not been achieved since there is no immediate indication of vein entry in the see-through chamber. The phlebotomist may therefore unnecessarily repeat the venipuncture procedure, requiring replacement of the evacuated container and/or the needle assembly itself. Such a repetitive process prolongs the physical and emotional discomfort endured by the patient. In such cases, a phlebotomist may use a blood collection set to provide some entry indication, and will then incur the cost of the blood collection set, as well as the cost of a discard tube.

It would therefore be desirable to provide an improved blood collection device that permits blood flow through a relatively short needle directly into a flashback chamber, thereby providing immediate indication of successful vein entry.

SUMMARY OF THE INVENTION

The invention, which is defined in claim 1, provides a needle assembly for the extraction of at least one fluid sample into an evacuated container for laboratory testing. The needle assembly provides a clear or translucent housing with sufficient dead space for blood to flow into a flashback chamber for visualization by the user to confirm successful vein entry, with an internal vent mechanism.

In one embodiment, the invention relates to a needle assembly comprising a housing defining a housing interior, a cannula having a patient puncture tip extending from a first end of the housing, and a non-patient puncture tip extending from a second end of the housing. The non-patient puncture tip and the patient puncture tip are in fluid communication with each other through the cannula, such that the sole communication path between the housing interior and the external environment is via the patient puncture tip. A porous vent is positioned within the housing interior to separate the housing interior into a first chamber and a second chamber, with the cannula being in fluid communication with the first chamber. The porous vent includes pores for passage of blood therethrough from the first chamber to the second chamber. The first chamber and the second chamber are configured such that upon insertion of the patient needle tip into a patient, blood flows through the cannula and into the first chamber without sealing the porous vent. Upon application of an evacuated container to the non-patient puncture tip, blood is drawn from the first chamber and air is drawn from the second chamber, thereby establishing a negative pressure within the second chamber with respect to an external environment of the needle assembly. Blood can thereafter be drawn into the first chamber and through the porous vent, with a negative pressure maintained in the second chamber.

In one embodiment, the cannula includes a first end comprising the patient puncture tip and a second end comprising the non-patient puncture tip, with an opening between the first end and the second end providing fluid communication between the cannula and the first chamber of the housing. In an alternate embodiment, the cannula comprises a first cannula having a patient puncture tip, with the needle assembly further comprising a second cannula including the non-patient puncture tip, with the first cannula and the second cannula substantially axially aligned and separated by a gap in fluid communication with the first chamber of the housing. A sleeve may also extend about the non-patient puncture tip.

In a particular embodiment, the first end of the housing comprises an elongate longitudinal first portion having a first diameter and the second end of the housing comprises a second portion having a second diameter larger than the first diameter of the first portion. In such an embodiment, the porous vent may be positioned within the housing interior between the first portion having a first diameter and the second portion having a second diameter. Alternatively, the porous vent may be positioned within the housing interior at a location spanning the transition between the first diameter of the first position and the second diameter of the second position.

In yet a further embodiment, a method of preventing leakage of blood from a needle assembly is provided. The method involves receiving blood through a patient puncture tip and into a first chamber of a needle assembly, with the needle assembly including a needle housing defining a housing interior; a cannula having the patient puncture tip extending from a first end of the needle housing; a non-patient puncture tip extending from a second end of the needle housing, the non-patient puncture tip and the patient puncture tip being in fluid communication with each other through the cannula; and a porous vent positioned within the housing interior and separating the housing interior into a first chamber and a second chamber. The cannula is in fluid communication with the first chamber such that the sole communication path between the housing interior and the external environment is via the patient puncture tip, and the porous vent includes pores for passage of blood therethrough from the first chamber into the second chamber. Fluid communication is established between the non-patient puncture tip and an evacuated collection container, such that blood contained within the first chamber is drawn into the evacuated collection container and air is drawn out of the second chamber through the porous vent. As such, a negative pressure is established within the second chamber relative to the external environment of the needle assembly, such that blood flows through the cannula into the first chamber and contacts the porous vent. Blood is then drawn through the pores of the porous vent toward the second chamber such that after removing the patient puncture tip from the vasculature of the patient any blood contained within the cannula is displaced away from the patient puncture tip based upon the negative pressure established within the second chamber.

Additionally, a further step may include establishing fluid communication between the non-patient puncture tip and a second evacuated collection container prior to drawing blood through the patient puncture tip and through the cannula into the second evacuated collection container, followed by releasing the fluid communication between the non-patient puncture tip and the second evacuated collection container.

The invention is directed to a method of collecting a sample of blood from a patient into an evacuated blood collection tube using a blood collection assembly having a patient needle tip and a non-patient needle tip and a housing having a flashback visualization chamber. The method involves using a needle assembly comprising a housing having a porous vent positioned therein to separate an interior of the housing into a first chamber forming the flashback visualization chamber and a second chamber, the first chamber and second chamber being configured such that air is drawn out of the second chamber through the porous vent and into the evacuated blood collection tube along with the blood sample, thereby establishing a negative pressure within the second chamber. The negative pressure causes blood to be drawn into the first chamber and contact the porous vent, such that after the patient needle tip is removed from the patient, the negative pressure within the second chamber draws blood from the patient needle tip toward the second chamber, thereby preventing leakage of blood from the patient needle tip.

DESCRIPTION OF THE DRAWINGS

FIG. 1 is a cross-sectional view of a typical configuration of the needle assembly.
FIG. 2 is a cross-sectional view of a second configuration.
FIG. 3 is a cross-sectional view of a third configuration.
FIG. 4 is a cross-sectional view of a fourth configuration.
FIG. 5 is a schematic view of the needle assembly of FIG. 1 prior to use.
FIG. 6 is a schematic view similar to FIG. 5, but showing the first sign of venous entry.
FIG. 7 is a schematic view of a fifth configuration.
FIG. 8 is a perspective view of a needle assembly having a flash chamber in a further embodiment.
FIG. 9 is a rear perspective view of the needle assembly having a flash chamber of FIG. 8.
FIG.10 is an exploded view of the needle assembly having a flash chamber of FIG. 8.
FIG. 11A is a cross-sectional view of the needle assembly having a flash chamber of FIG. 8.
FIG. 11B is an enlarged cross-sectional view of a portion of the needle assembly of FIG. 11A.
FIG. 12A is a cross-sectional view of a needle assembly having a flash chamber used in connection with a blood collection assembly in yet a further embodiment.
FIG. 12B is an enlarged sectional view of a portion of the needle assembly of FIG. 12A.

DETAILED DESCRIPTION

The invention provides a needle assembly for blood collection that provides a visual indication of vein entry ("flashback") upon collection of a blood or other fluid sample from a patient into one or more evacuated blood collection tubes and inhibits leakage of the blood or fluid sample from the IV cannula on removal from the patient.

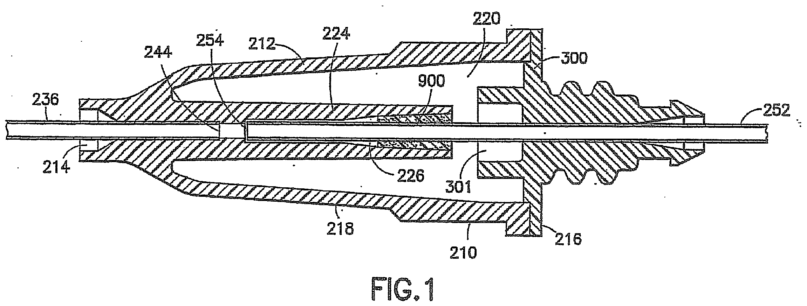

Various configurations are shown in **FIGS. 1-7.** With reference to **FIG. 1,** this embodiment is directed to a needle assembly **210** with a housing **212** having a fluid inlet end **214,** a fluid outlet end **216** and a frustum-shaped exterior wall **218** extending between the ends. Exterior wall **218** defines the housing interior **220.** Housing **212** further includes a cylindrical interior wall **224** that extends in the housing interior **220** from fluid inlet end **214** substantially concentrically with cylindrical exterior wall **218** to a vent plug **900.** Cylindrical interior wall **224** and vent plug **900** define a flashback chamber **226.**

Needle assembly **210** also includes a fluid inlet cannula **236** having an exterior end that defines a sharpened bevel and an interior end **244** that is mounted fixedly in fluid inlet end **214** of housing **212.** Fluid inlet cannula **236** is characterized further by a substantially cylindrical lumen extending between the ends and communicating with the interior of housing **212.**

Needle assembly **210** further includes a fluid outlet cannula **252.** Outlet cannula 252 concludes a blunt interior end **254,** an exterior end defining a sharpened bevel and a substantially cylindrical lumen extending between the ends. Portions of outlet cannula **252** between the ends are securely affixed in outlet end **216** of housing **212.** Outlet cannula **252** is mounted so that interior end **254** passes substantially coaxially into interior wall **224** and so that interior end **254** of outlet cannula **252** substantially aligns axially with interior end **244** of inlet cannula **236.** Additionally, interior end **254** of outlet cannula **252** is spaced only a small distance from interior end **244** of inlet cannula **236.** An axial gap between interior end **254** of outlet cannula **252** and interior end **244** of inlet cannula **236** that is less than 0.5mm may result in a flashback that is inconsistent.

Cylindrical interior wall **224** is dimensioned relative to outlet cannula **252** to achieve both desirable flow of blood through assembly **210** and to achieve effective flashback indication. In particular, cylindrical interior wall **224** preferably is dimensioned to provide a radial gap around outlet cannula **252** of about 0.2mm, as indicated by dimension "c" in **FIG. 1.** This gap achieves a substantially laminar blood flow within flashback chamber **226** and prevents blood hemolysis. Additionally, the small radial gap between cylindrical inner wall **224** and outlet cannula **252** enables a drop of blood to be spread thinly across the radial gap in flashback chamber **226** to provide a magnified flashback indication with a very small volume of blood. Thus, an easily visualized flashback indication is achieved quickly at the first appearance of blood from interior end **244** of inlet cannula **236.**

Needle assembly **210** further includes a sealable sleeve **261** mounted to fluid outlet end **216** of housing **212** and covering exterior end **258** of outlet cannula **252** when sealable sleeve **261** is in an unbiased condition. However, sealable sleeve **261** can be collapsed in response to pressure exerted by the stopper of an evacuated tube for urging exterior end **260** of outlet cannula **252** through both sealable sleeve **261** and stopper of an evacuated tube, as known in the art.

The above configuration is described in terms of a vent plug. However, any vent mechanism is suitable. The vent mechanism may be, for example, a porous vent plug formed from a matrix or carrier material, typically hydrophobic, that is coated with, impregnated with, or otherwise, contains a hydrophilic material that swells on contact with aqueous or water containing substances. The hydrophobic carrier material can be but is not limited too, high-density polyethylene, polytetrafluoroethylene, ultra-high molecular weight polyethylene, Nylon 6, polypropylene, polyvinylidine fluoride and polyethersulfone. The swellable nature of the hydrophilic material thereby provides the sealing function in the vent upon contact with blood. It is also possible to use a porous vent plug that becomes sealed upon contact with blood using biological phenomena, e.g., by clotting and/or cell agglutination that blocks the vent; a superabsorbant material to seal the vent by swelling on contact with an aqueous fluid; or a one-way valve, (e.g., a thin flap such as plastic film covering a vent, a deformable seal such as a rubber or plastic duckbill valve, or a deformable wrap over a vent). It should be noted that any combination of these various mechanisms is also possible.

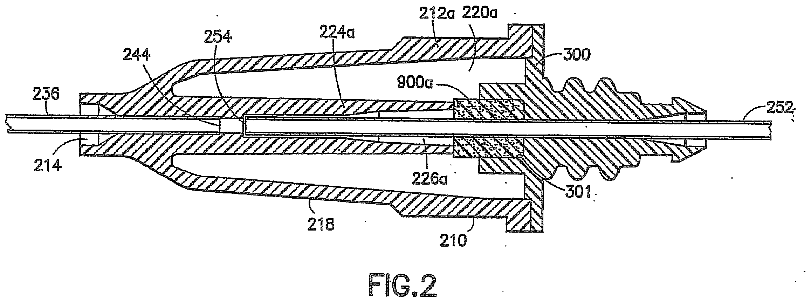

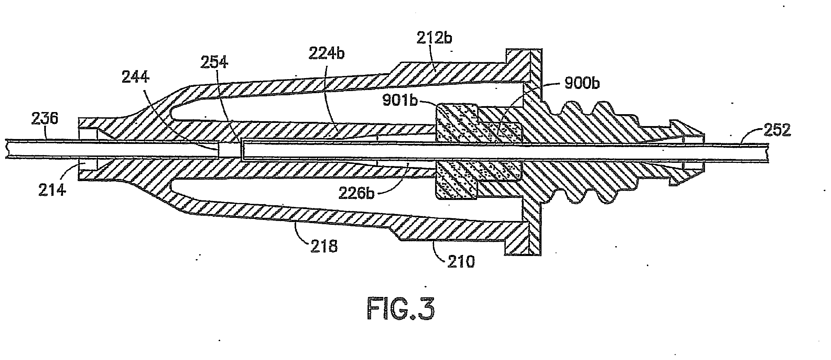

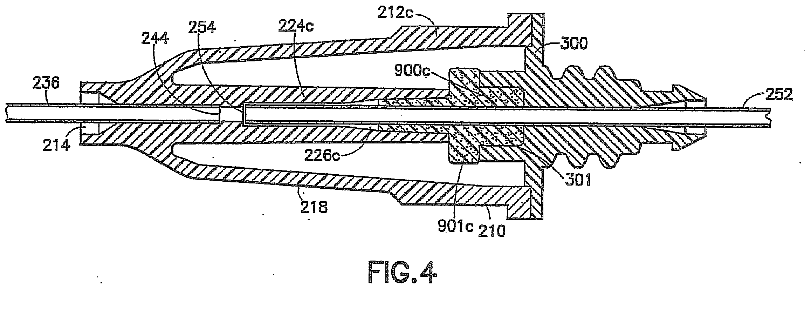

**FIGS 2-4** show configurations with varying vent plugs. **FIG. 2** shows a vent plug **900a,** which is located at the end of the cylindrical inner wall **224a** and fitted into a recess **301** in the housing interior non-patient wall **300.** **FIG. 3** shows a vent plug in a similar location to that of **FIG. 2,** however, vent plug **900b** has a shoulder **901b.** **FIG. 4** shows a vent plug **900c** that is located both within the cylindrical inner wall **224c** and the recess **301** in the housing interior non-patient wall **300,** and has a shoulder **901c.** The vent plug location in each of these configurations is such that no air can flow out of the flashback chamber **226** into the housing interior **220** without passing through the vent mechanism **(900 a, b, c).**

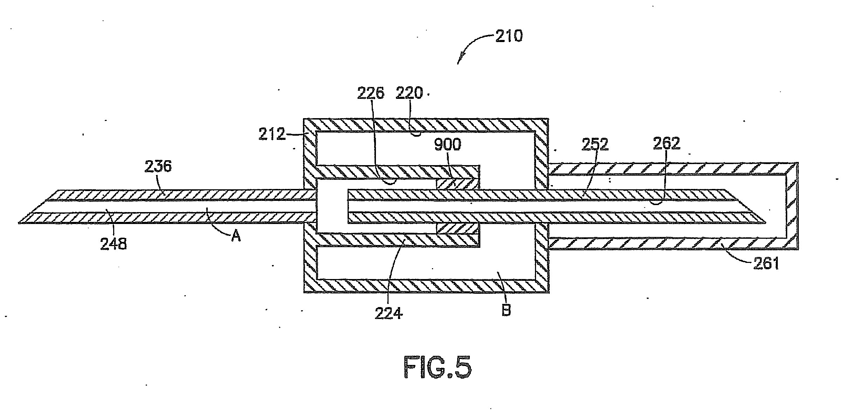

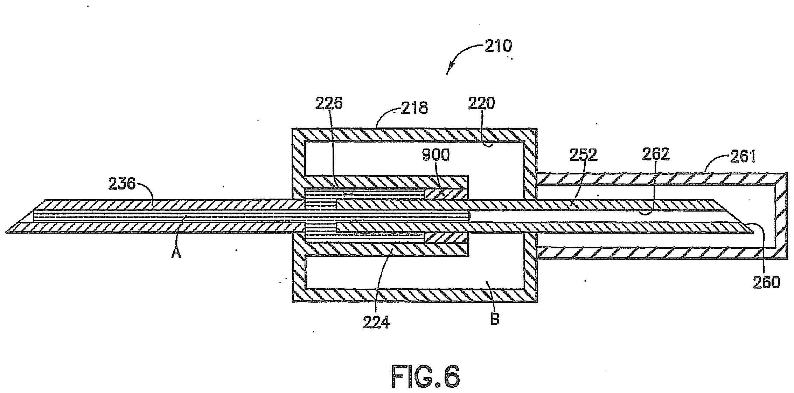

**FIGS. 5** and **6** provide schematic representations of the needle assembly **210** of **FIG. 1** before and after a conventional venipuncture, in which, the needle assembly **210** is connected to a holder (not shown) and punctures the patient's skin to make a vein entry. Upon vein entry, blood enters the IV cannula **236** and flows toward the flashback chamber **226.** The blood flows from inlet cannula **236** into the space between inlet and outlet cannula, such that blood flows both into the outlet cannula **252** and into flashback chamber **226.** At this point in time, flashback chamber **226** indicates successful vein entry and reduces the volume of air present in housing **212** shown in **FIG. 6.** Air that was at atmospheric pressure within the lumen of the IV cannula **248,** flashback chamber **226,** housing interior **220,** and the lumen of the non-patient cannula **262** prior to vein entry, thus experiences compression due to the influence of venous pressure and this air is therefore forced through the IV cannula **236** shown in **FIG. 6** into the flashback chamber **226** and through the vent plug into chamber **220.** Blood flow into housing interior **220** is prevented by the vent plug **900,** which allows the pressurized air to flow through it, but seals on contact with blood, thereby trapping the compressed air (at venous pressure) in housing interior **220.** Blood flow in the entire needle assembly ceases once the pressure within chamber **226** and the venous pressure are equal.

Once the steps set forth in the previous paragraph occur, and venous entry is visually confirmed by the phlebotomist, an evacuated container (not shown), is then inserted into the holder such that exterior end **260** of second cannula **252** penetrates the stopper of the container, as known in the art. Upon penetration of the stopper by second cannula **252,** a negative pressure gradient is transmitted to chamber **226,** causing blood to flow from chamber **226** into the container.

The needle assemblies described above desirably should be small for convenient use, but should be constructed to ensure reliable and rapid flashback. The occurrence of flashback in the needle assemblies described and illustrated above operate pursuant to the ideal gas law. In particular, at very low densities all gases and vapors approach ideal gas behavior and closely follow the Boyle's and Charles' laws given by:

$$P_1 V_1 = P_2 V_2$$

where:

> $P_1$ denotes the pressure of air within the needle assembly before needle insertion;
>
> $P_2$ denotes the pressure of air within the needle assembly after vein entry;
>
> $V_1$ denotes the volume of air within the needle assembly before vein entry; and
>
> $V_2$ denotes the volume of air within the needle assembly after vein entry.

Design parameters should keep the needle device as small as possible for easy use, while ensuring an appropriate volume as specified by the preceding equation. **FIGS. 5** and **6** provide schematic representations of the needle assembly **210** of **FIG. 1** for purposes of depicting the application of the ideal gas law. In this regard, **A** identifies the volume of lumen **248** through inlet cannula **236. B** denotes the total volume of the housing interior **220,** flashback chamber **226,** lumen **242** through outlet cannula **252** and sealable sleeve **261.** Referring again to the preceding equation, $P_1$ is the pressure within needle assembly **210** before use, and hence substantially equals atmospheric pressure. Atmospheric pressure will vary slightly from time to time and from location to location. However, for purposes of this analysis, atmospheric pressure $P_1$ will be assumed to be 760mm Hg. $P_2$ in the preceding equation is the volume of the dead space in needle assembly **210** after vein entry. More particularly, after vein entry, blood will fill lumen **248** of inlet cannula **236,** thereby reducing the volume to be occupied by gas in remaining portions of needle assembly **210** and hence increasing the pressure of air in the remaining portion of needle assembly **210.** A needle assembly with dimensions approximately as shown in **FIG. 1** will have a pressure $P_2$ of about 790mm Hg at venous pressure (with tourniquet). $V_1$ in the preceding equation defines the volume of the total dead spaced in needle assembly **210** before use, and hence will equal **A + B** as shown in **FIG. 5.** $V_2$ defines the dead space in the device after vein entry, and with lumen **248** of inlet cannula **236** filled with blood. Hence, $V_2$ in the preceding equation will equal **B.** These input parameters can be employed to define a minimum desired size for the respective components of needle assembly **200** as shown in the following application of the ideal gas law equation.

$$P_1 V_1 = P_2 V_2$$

$$P_1/P_2 = V_2/ V_1$$

$$760/790 = B/(A+B)$$

$$0.962 = B/(A+B)$$

$$0.962(A+B) = B$$

$$0.038B = 0.962A$$

$$B=25.3A$$

Therefore, dead space in housing **212,** outlet cannula **252** and sleeve **261** advantageously is at least 25.3 times the volume defined by lumen **248** through inlet cannula **236,** and most advantageously is about 26 times the volume of lumen **248.** However, other configurations are possible and will function as described herein.

The immediate response when an evacuated tube is placed in communication with outlet cannula **252** is to draw blood from the vein into tube (not shown). The highest-pressure gradient is always maintained between the vein and the evacuated tube. An axially aligned inlet cannula **236** and outlet cannula **252,** therefore provide an unobstructed path for blood flow from the vein into evacuated tube.

When the requisite tubes are filled with blood, the needle assembly is removed from the vein. The sealed nature of the vent plug **900** inhibits the pressurized air within housing interior **220** from then moving into the flashback chamber **226** and into the inlet cannula **236,** which could promote dripping of blood from the IV cannula tip.

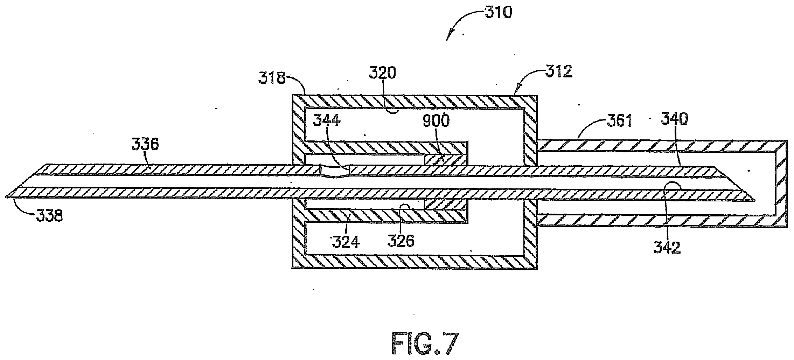

The preceding configurations show structurally separate inlet and outlet cannulas that are axially aligned with one other and placed in close end-to-end relationship with one another. However, the principals of the invention described above also can be achieved with a single cannula formed with a transverse slot or aperture within the flashback chamber. For example, **FIG. 7** schematically shows a needle assembly **310** with a housing **312** that is substantially identical to housing **212** described and illustrated above. Needle assembly **310** differs from needle assembly **210** in that a single double end needle cannula **336** is provided and passes entirely through housing **312.** More particularly, needle cannula **336** includes a venous entry end **338,** a non-patient end **340** and a lumen **342** extending therebetween. Portions of cannula **336** within inner wall **324** include a slot or aperture **344** to provide communication between lumen **342** and flashback chamber **336** within inner wall **324.** Needle assembly **310** functions substantially in the same manner as needle assembly **210** described and illustrated above.

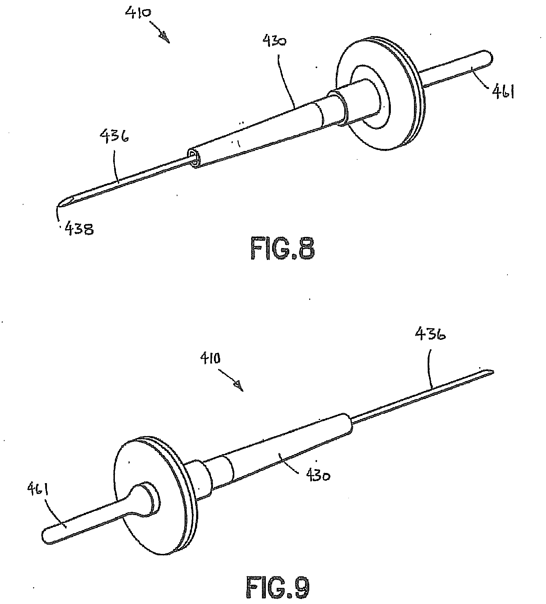

**FIGS. 8-11** depict a needle assembly of the invention. In certain embodiments of the needle assembly described with respect to **FIGS. 1-7,** the housing interior includes a vent plug **900,** which seals the flashback chamber **226/326** from the housing interior **220/320.** In such previously described embodiments, the vent plug is described as sealing upon flow of blood into the flashback chamber, thereby inhibiting any pressurized air that may build up within the housing chamber **220/320** (such as upon displacement of air from the flashback chamber **226/326** into the housing chamber **220/320** during the initial flash procedure) from moving in a reverse direction toward the inlet cannula. In the embodiment of **FIGS. 8-11,** a porous vent is positioned within the housing at a location such that the vent divides the housing into two chambers having sizes and dimensions to establish predetermined volumes thereto. Moreover, the porous vent remains porous to blood and does not seal upon contact with blood. Desirably the blood does not contact the porous vent at the initial flash indication, but such contact occurs at a later point during use of the assembly, as will be described in more detail herein.

For example, **FIGS. 8-11** show a needle assembly **410** similar to that described in connection with **FIG. 1-6** above. As shown in **FIGS. 8-11,** needle assembly **410** includes a housing **412** having a fluid inlet end or first end **414** and a fluid outlet end or second end **416.** Needle assembly **410** includes exterior wall **418** defining the housing interior. Exterior wall **418** extends generally longitudinally at the first end **414** forming an elongate longitudinal first portion **419** having a first diameter. At second end **416,** exterior wall **418** forms a second portion **421** that has a second diameter that is generally larger than the first diameter of the first portion **419.** Accordingly, housing **412** may form a structure having a generally T-shaped cross-section. The exterior wall **418** at second end **416** may be a separate element **428** that is attachable to main body portion **430** forming housing **412,** thereby assisting in manufacture and assembly of needle assembly **410.** First portion **419** and second portion **421** may be arranged relative to each other in a variety of arrangements, so long as they are capable of functioning for transport of air therebetween as discussed herein.

Needle assembly **410** further includes a fluid inlet cannula **436** extending from first end **414** of housing **412.** Fluid inlet cannula **436** includes an exterior end **439** that defines a sharpened bevel at patient puncture tip **438,** and extends within first end **414** of housing **412,** and may be fixedly mounted therein. Fluid inlet cannula **436** is characterized further by a substantially cylindrical lumen extending between the ends and communicating with the interior of housing **412.**

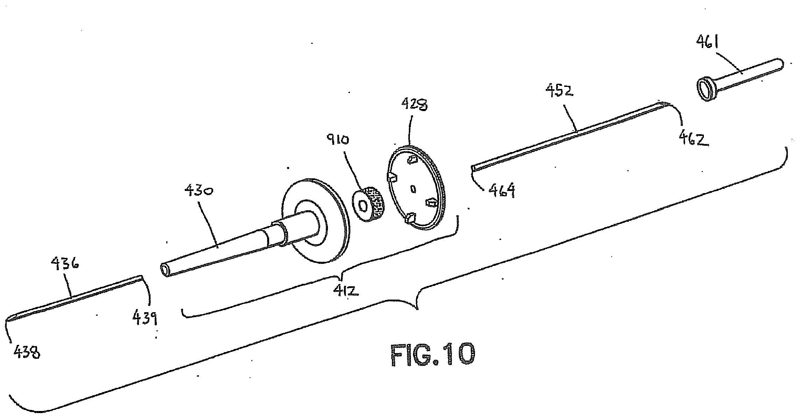

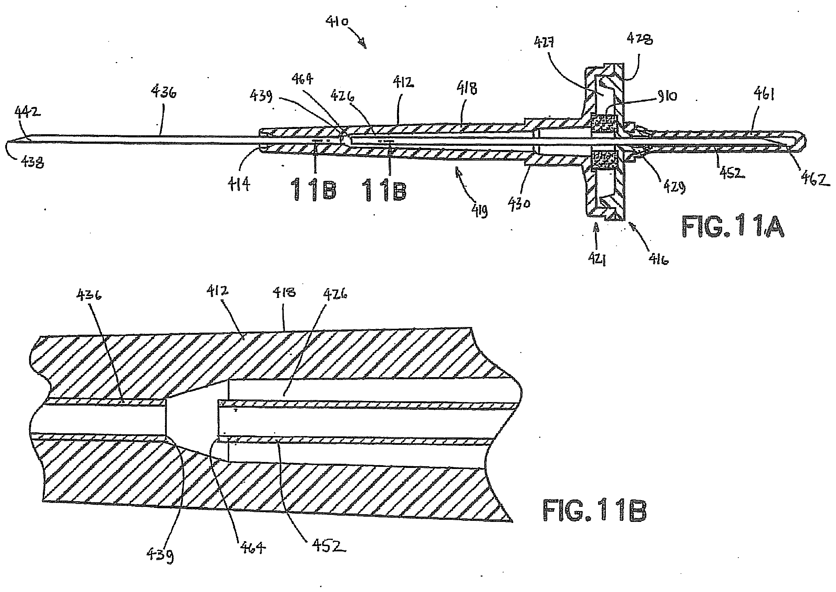

Needle assembly **410** also includes a non-patient puncture tip extending from second end **414** of housing **412.** As seen in **FIG. 10,** this may be accomplished by providing needle assembly **410** with a second cannula in the form of fluid outlet cannula **452.** In particular, the end of fluid outlet cannula **452** may define a sharpened bevel forming non-patient puncture tip **462.** Fluid outlet cannula **452** extends within second end **416** of housing **412,** and may be fixedly mounted therein. Fluid outlet cannula **452** is characterized further by a substantially cylindrical lumen communicating with the interior of housing **412.** Outlet cannula **452** is mounted within housing **412** so that an interior end **464** passes substantially coaxially therein such that outlet cannula **452** substantially aligns axially with the interior end of inlet cannula **436.** Desirably, this is achieved by mounting outlet cannula **452** at a location adjacent second end **416** of housing **412,** such that the interior end **464** of outlet cannula **452** extends within housing **412** to a location adjacent the interior end **439** of inlet cannula **436.** As seen in FIG. 11B, the interior end **464** of outlet cannula **452** is spaced only a small distance from the interior end **439** of inlet cannula **436,** thereby forming an axial gap therebetween for flow of blood into flashback chamber **426** about outlet cannula **452.** The distance between the interior end **464** of outlet cannula **452** and the interior end **439** of inlet cannula **436** forming the axial gap is sufficient to provide for flow of blood into flashback chamber **426** based upon the patient's blood pressure after venipuncture. In certain embodiments, an axial gap that is less than 0.5mm may result in a flashback that is inconsistent.

As seen in **FIG. 11B,** fluid inlet cannula **436** and fluid outlet cannula **452** are positioned and dimensioned within housing **412** so as to achieve both desirable flow of blood through assembly **410** and to achieve effective flashback indication. In particular, wall **418** of housing **412** is dimensioned to provide a radial gap around outlet cannula **452** of about 0.2mm at an area surrounding the internal end **464** thereof. This gap achieves a substantially laminar blood flow within flashback chamber **426** and prevents blood hemolysis. Additionally, the small radial gap between the inner surface of wall **418** and outlet cannula **452** at the area surrounding the internal end **464** enables a drop of blood to be spread thinly across the radial gap in flashback chamber **426** to provide a magnified flashback indication with a very small volume of blood. Thus, an easily visualized flashback indication is achieved quickly at the first appearance of blood within flashback chamber **426.** It is contemplated that internal end **464** of outlet cannula **452** may be partially supported within housing **412,** so long as blood flow into flashback chamber **426** is achieved about the internal end **464.**

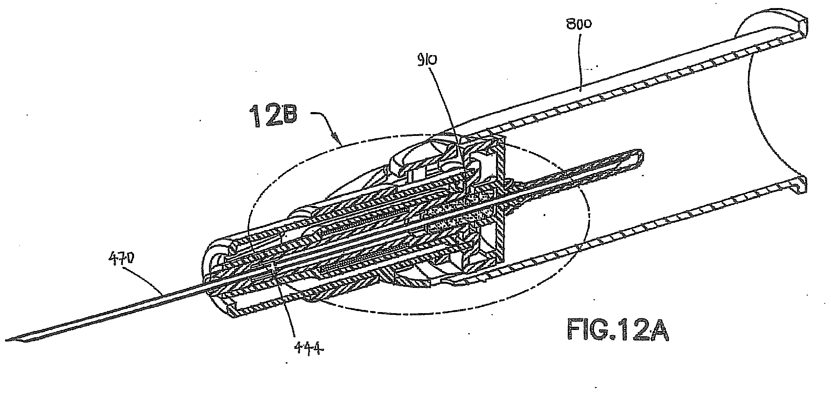

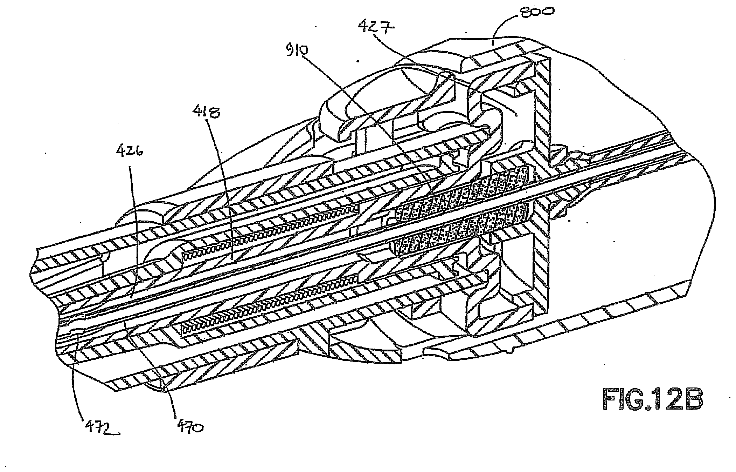

In an alternate arrangement, a single cannula is provided, similar to that embodiment discussed in connection with **FIG. 7.** Such an arrangement is depicted in the embodiment of **FIG. 12A** and **12B** (shown in connection with a blood collection assembly as will be described in more detail herein). In such an arrangement, the fluid inlet cannula and the fluid outlet cannula represent one single cannula **470,** having a patient puncture tip **438** a non-patient puncture tip **462,** and a lumen **442** extending therethrough, and with the body of the cannula **470** being fixedly attached to a portion of the housing **412** and passing entirely through housing **412.** A portion of cannula **470** extending through housing **412** includes one or more openings such as a slot or aperture **444** to provide communication between lumen **442** and flashback chamber **436** within housing **412.** In the embodiment seen in **FIGS. 12A** and **12B,** two separate apertures are shown on opposing sides of cannula **470,** although it is contemplated that any number of such openings can be included to provide for blood flow into flashback chamber **436.**

Returning to the embodiment of **FIGS. 8-11,** needle assembly **410** further includes a sealable sleeve **461** mounted to fluid outlet end **416** of housing **412.** This may be accomplished by providing a mounting protrusion **429** at second end **416** of housing **412,** such as on element **428,** with sealable sleeve **461** representing an elastomeric element that can be frictionally fit or otherwise affixed over protrusion **429.** Sealable sleeve **461** covers non-patient puncture tip **462** at the exterior end of outlet cannula **452** when sealable sleeve **461** is in an unbiased condition. However, sealable sleeve **461** can be collapsed in response to pressure exerted by the stopper of an evacuated tube for urging exterior end **460** of outlet cannula **452** through both sealable sleeve **461** and the stopper of an evacuated tube, as known in the art.

The embodiment of **FIGS. 8-11** further includes a porous vent **910** positioned within the interior of housing **412.** Porous vent **910** is positioned within housing **412** to divide housing **412** into two distinct chambers, namely, a first chamber represented by flashback chamber **426** and a second chamber represented by secondary chamber **427.** Porous vent **910** may be constructed of a suitable material as described above with respect to vent plug **900,** albeit without the hydrophilic material that swells on contact. In this manner, porous vent **910** is adapted to vent air therethough, and represents a porous structure including a plurality of pores that allow for passage of blood therethrough. As discussed in more detail herein, during use of needle assembly **410,** the internal pores within porous vent **910** at least partially fill with blood due to the negative pressure established within secondary chamber **427.** Such filled pores in combination with the negative pressure within secondary chamber **427** prevent air flow between the secondary chamber **427** and the flashback chamber **426,** and provide for fluid resistance of the blood flow through porous vent **910,** as will be described in further detail.

Desirably, porous vent **910** is positioned within the interior of housing **412** between first portion **419** and second portion **421.** In this manner, first portion **419** of housing **412** essentially defines the flashback chamber **426,** and second portion **421** of housing **412** essentially defines the secondary chamber **427.** Alternatively, porous vent **910** may be positioned within the interior of housing **412** at a location spanning the transition between the first diameter of first portion **419** and the second diameter of second portion **421,** as shown in the embodiment of **FIGS. 12A** and **12B.** In any event, porous vent **910** is generally a cylindrically-shaped member with a central opening therein axially encircling a portion of the cannula, particularly fluid outlet cannula **452.**

The interior volume of housing **412** is defined by the sum of the volumes of flashback chamber **426** and secondary chamber **427** as well as the volume represented by the pores of porous vent **910.** Such interior volume is configured so as to provide for certain attributes to the needle assembly **410,** in particular with respect to the ability of the secondary chamber **427** to be at least partially evacuated of a portion of the air therein to establish a negative pressure therein upon application of an evacuated tube to needle assembly **410** during use thereof. Such negative pressure within secondary chamber **427** draws blood through the pores of porous vent **910** based on when blood contacts porous vent **910** and partially fills the pores thereof. In a particular embodiment of the invention, the overall interior volume of housing **412** may be from about 300 mm$^3$ to about 400 mm$^3$. Such a volume is particularly useful for the intended use of needle assembly **410** for conventional venipuncture for drawing a blood sample from a patient using a needle cannula having a conventional gauge for venipuncture as is known in the art. With such an internal volume, porous vent **910** is desirably positioned within housing interior so as to define flashback chamber **426** as having a volume that represents from about 5 percent to about 20 percent of the total overall volume of housing **412,** desirably from about 7 percent to about 12 percent of the total overall volume of housing **412,** including the volume of secondary chamber **427** and the volume of the pores within porous vent **910.** Such a ratio of the flashback chamber **426** to the total overall volume of the housing **412** assures that flashback chamber **426** has sufficient volume to properly visualize the initial flash, and desirably while preventing blood from fully contacting the porous vent **910** at initial venipuncture, based on the initial build-up of pressure within secondary chamber **427** caused by venous pressure forcing the blood into flashback chamber **426.** Such volume ratios are effective for the intended use as described in further detail herein, wherein blood flowing into flashback chamber **426** upon initial venipuncture does not contact porous vent **910,** and wherein at least a portion of the air is drawn out from secondary chamber **427** based upon application of an evacuated blood collection tube to the needle assembly **410.** In this manner, secondary chamber **427** can effectively draw blood from within flashback chamber **426** and from within fluid inlet cannula **426** toward secondary chamber **427,** such as into and through porous vent **910,** when patient puncture tip **438** is removed from the patient and is exposed to the external environment. In one particular embodiment, the total interior volume of the housing **412** is about 380 mm$^3$, with the flashback chamber **426** having a volume of about 30 mm$^3$, the secondary chamber **427** having a volume of about 300 mm$^3$, and the pores of the porous vent **910** representing a volume of about 50 mm$^3$.

Needle assembly **410** may be assembled as follows. Fluid inlet cannula **436** is positioned through first end **414** of housing **412** such that the open interior end **439** is positioned within an interior portion of housing **412** at first portion **419** and patient puncture tip **438** extends externally of first end **414.** Fluid outlet cannula **452** is positioned within housing **412** through the opposite end, such that open internal end **464** is positioned within an interior portion of housing **412** at first portion **419** adjacent interior end **439** of fluid inlet cannula **436,** with a slight gap therebetween, and with non-patient puncture tip extending externally of second end **416.** Fluid inlet cannula **436** and fluid outlet cannula **452** may be affixed therein in any known manner, desirably through a medical grade adhesive.

In alternate embodiments including only a single cannula **470,** such cannula **470** is affixed within housing **412** such that opening **472** is positioned within the interior of housing **412** at first portion **419,** with patient puncture tip **438** extending externally of first end **414** and non-patient puncture tip **462** extending externally of second end **416.**

Porous vent **910** is then inserted within housing **412** and positioned over fluid outlet cannula **454** (or over the single cannula **470**), and element **428** is thereafter affixed to the second end **416,** enclosing the interior of housing **412.** Sealable sleeve **461** is then affixed over protrusion **429.** As such, the interior of housing **412** is closed from the external environment, with the sole path for fluid communication between the interior of housing **412** and the external environment being provided through the patient puncture tip **438.**

Needle assembly **410** assembled as such can be used in connection with a blood collection tube holder **800,** as depicted in the embodiment shown in **FIG. 12.** Such assembly may be accomplished through the rear open end of blood collection tube holder **800,** so that the entire needle assembly **410** is inserted to a portion where at least patient puncture tip **438** and at least a portion of inlet cannula **436** extend out through the front end of blood collection tube holder **800.** In embodiments where second portion **421** of needle assembly **410** is radially larger than first portion **419,** such an insertion and arrangement enables the secondary chamber **427** to be fully contained within the internal space within collection tube holder **800,** and with flashback chamber **426** extending out from a front end thereof.

In use, needle assembly **410** may be provided with collection tube holder **800** attached thereto. Patient puncture tip **438** is inserted through the skin of a patient and into the patient's vasculature, desirably into a vein. Upon venipucture, a closed environment is achieved within housing **412,** since housing **412** is an entirely closed structure, and since sealable sleeve **461** closes off the only outlet of housing **412** (i.e., fluid outlet cannula **452**). The patient's blood pressure causes blood to flow through patient puncture tip **438,** into fluid inlet cannula **436,** and out interior end **439** (or through opening **472** in the embodiment of **FIG. 12**), into flashback chamber **426** surrounding interior end **464** of outlet cannula **452.** The transparent or translucent nature of housing **412** permits visualization of the blood within flashback chamber **426,** providing an indication that venipuncture is achieved.

Since the interior of housing **412** is a closed environment, the flow of blood into flashback chamber **426** causes air to be trapped within the housing interior, including within flashback chamber **426,** porous vent **910** and secondary chamber **427,** as well as within fluid outlet cannula **452,** causing such trapped air to be slightly pressurized therein. Flashback chamber **426** and secondary chamber **427** are configured through their size and dimensions such that the volumes thereof permit blood to flow into flashback chamber **426** at this initial venipucture, but the build up of air pressure within the pores of porous vent **910** and within secondary chamber **427** prevents blood from fully contacting porous vent **910,** and desirably prevents blood from even partially contacting porous vent **910** at the initial venipuncture.

After such initial venipuncture and flash visualization, a sample collection container having a negative pressure therein, such as an evacuated blood collection tube (not shown) as is commonly known in the art, is inserted within the tube holder **800.** The stopper (not shown) of such evacuated container contacts and displaces sealable sleeve **461,** causing non-patient puncture tip **462** to puncture through sealable sleeve **461** and through the stopper of the evacuated container. At this point, fluid communication is established between the non-patient puncture tip **462** and the interior of the evacuated collection container. The negative pressure within the evacuated collection container draws the blood that has collected within flashback chamber **426** into fluid outlet cannula **452** and into the evacuated collection container. Along with the blood within flashback chamber **426,** the negative pressure within the evacuated collection container will also draw at least a portion of the air out of the flashback chamber **426** and out of the secondary chamber **427** through the pores of porous vent **910,** toward and into the evacuated collection container. In addition, the close proximity and alignment of fluid outlet cannula **452** and fluid inlet cannula **426** causes blood to be drawn from fluid inlet cannula **436** and from the patient, simultaneously with such air being drawn from the flashback chamber **426** and secondary chamber **427.**

Such drawing of air reduces the pressure within the flashback chamber **426** and the secondary chamber **427,** establishing a negative pressure therein with respect to the patient's bloodstream and with respect to the external environment. This negative pressure that has been established within the interior of housing **412,** and specifically within flashback chamber **426** and secondary chamber **427,** draws additional blood from within fluid inlet cannula **436** and from the patient into flashback chamber **426,** with the blood contacting porous vent **910.** With such blood filling flashback chamber **426,** the blood fully contacts the surface of porous vent **910** that extends within flashback chamber **426,** and begins to fill the pores of porous vent **910.** Such filling of the pores of porous vent **910** that are directly at the interface of porous vent **910** and flashback chamber **426** closes off the porous vent from airflow therethrough, but does not fully act as a seal, in that the blood does not cause the material of the porous vent to swell or close off to air flow, but instead merely physically fills the voids within the porous vent. Moreover, since a portion of the air within secondary chamber **427** has been drawn out from secondary chamber **427,** secondary chamber **427** represents a closed chamber with a negative pressure therein relative to the external environment. Secondary chamber **427** will therefore continue to have a drawing effect on the blood within the pores of porous vent **910** and within flashback chamber **426** through the pores of porous vent **910** toward secondary chamber **427,** without releasing any air from the secondary chamber **427** in the opposite direction due to the pores of porous vent **910** at the interface of the flashback chamber **426** being filled with blood, thereby effectively preventing air flow through porous vent **910** due to the filled pores. The draw created by the negative pressure within secondary chamber **427** has a fluid resistance based on the blood filling the pores of porous vent **910** and based on the tortuous path created by the pores of porous vent **910,** and therefore is a gradual draw with reduced fluid movement.

At this point, the evacuated collection container and the secondary chamber **427** are both at a negative pressure with respect to the external environment (and with respect to the patient's bloodstream), and therefore both effect a draw from the fluid inlet cannula **436.** This mutual drawing effect may essentially establish an equilibrium within the flashback chamber **426,** such that the blood contained within the flashback chamber **426** is not drawn toward or into either the secondary chamber **427** through the pores of porous vent **910** or into the evacuated collection container through the fluid inlet cannula **436,** but instead essentially remains within flashback chamber **426** in a steady state. The negative pressure of the evacuated collection container draws blood directly from the patient through fluid inlet cannula **436,** due to the close proximity and alignment of fluid outlet cannula **452** and fluid inlet cannula **426,** as well as due to the equilibrium established within flashback chamber **426** (based on the opposite draw forces between the evacuated collection container and the evacuated secondary chamber **427**). The continual draw of blood into the evacuated collection container gradually causes the pressure within the collection container to increase.

Once the evacuated collection container is filled with the desired amount of blood, the container is removed from the non-patient puncture tip **462,** thereby releasing the fluid communication between the non-patient puncture tip **462** and the evacuated collection container, with sealable sleeve **461** then covering and closing off non-patient puncture tip **462.** Absent such draw from the negative pressure of the evacuated collection tube, the negative pressure within the secondary chamber **427** effects a slight draw on the blood within flashback chamber **426** through the pores of porous vent **910.** Such draw, however, is slow and gradual, due to the tortuous path of blood flow through the pores of porous vent **910.**

Additional evacuated collection containers can thereafter be inserted into tube holder 800 and used for sample collection through non-patient puncture tip **462** as described above, by placing a second evacuated collection container within the holder **800** and establishing fluid communication between the non-patient puncture tip **462** and the interior of the evacuated collection container by puncturing the stopper, as discussed. In such further sampling, the evacuated collection container and the secondary chamber **427** are both at a negative pressure, and therefore both effect a draw from the fluid inlet cannula. As above, this effect essentially establishes an equilibrium within the flashback chamber **426,** thereby preventing the blood contained within the flashback chamber **426** from being drawn toward or into either the secondary chamber **427** (through the porous vent **910**). The negative pressure of the evacuated collection container draws blood directly from the patient through fluid inlet cannula **436** as discussed above, due to the close proximity and alignment of fluid outlet cannula **452** and fluid inlet cannula **426.** Once any such additional evacuated collection containers are filled with the desired amount of blood, the container is removed from the non-patient puncture tip **462,** thereby releasing the fluid communication between the non-patient puncture tip **462** and the evacuated collection container, with sealable sleeve **461** then covering and closing off non-patient puncture tip **462.**

Once all of the desired blood samples have been drawn in this manner, patient puncture tip **438** is removed from the vasculature of the patient (i.e. from the bloodstream), thereby exposing the opening of patient puncture tip **438** to the external environment. Since the sole communication path between the housing interior and the external environment is through patient puncture tip **438,** the negative pressure established within secondary chamber **427** relative to the external environment will affect a gradual draw on the blood contained within flashback chamber **426** and within fluid inlet cannula **436** toward and through porous vent **910.** Such drawing effect will displace and move any blood contained within fluid inlet cannula **436** away from patient puncture tip **438,** toward secondary chamber **427,** thereby preventing any blood from leaking from patient puncture tip **438** out of fluid inlet cannula **436.** Such negative pressure within secondary chamber **427** may continue to have a gradual drawing effect through the porous vent **910** for a prolonged period of time after removal of patient puncture tip **438** from the patient, and may draw all of the remaining blood contained within fluid inlet cannula **436** and flashback chamber **426** through porous vent **910** and/or into secondary chamber **427.** Needle assembly **410** can then be properly disposed of in known manner.

The relative dimensional calculations, volumes and pressures apply to both illustrated and unillustrated embodiments of the invention.

**Claims**

1. A needle assembly comprising:

   a housing (412) defining a housing interior, said housing comprising a patient puncture tip (438) extending from a first end (414) of the housing and a non-patient puncture tip (462) extending from a second end (416) of the housing, the non-patient puncture tip and the patient puncture tip being in fluid communication with each other, wherein the sole communication path between the housing interior and an external environment is via the patient puncture tip; and
   a porous vent (910) positioned within the housing interior to separate the housing interior into a first chamber (426) and a second chamber (427) with the patient puncture tip and the non-patient puncture tip both in fluid communication with the first chamber of the housing, the porous vent including pores

the assembly **characterised in that** the pores are adapted for passage of blood therethrough form the first chamber to the second chamber, wherein the first chamber, the second chamber, and the porous vent are configured such that upon insertion of the patient puncture tip into a patient, blood flows into the first chamber without sealing the porous vent, and upon removal of at least a portion of air from the secondary chamber, blood is drawn from said first chamber, thereby establishing a negative pressure within said second chamber with respect to the external environment of the needle assembly.

2. The needle assembly of claim 1, further comprising a single cannula extending through the housing, said single cannula including a lumen extending therethrough, a first end comprising the patient puncture tip, a second end comprising the non-patient puncture tip, and an opening through the cannula into the lumen at a location between the first end and the second end providing fluid communication between the lumen of the cannula and the first chamber of the housing.

3. The needle assembly of claim 1, further comprising a first cannula extending from the housing and comprising the patient puncture tip, and a second cannula extending from the housing and comprising the non-patient puncture tip, the first cannula and the second cannula being substantially axially aligned within said housing interior and separated from each other by a gap in fluid communication with the first chamber of the housing.

4. The needle assembly of claim 1, further comprising a sealable sleeve extending about the non-patient puncture tip.

5. A method of preventing leakage of blood from a needle assembly comprising:

   a) receiving blood through a patient puncture tip and into a first chamber of the needle assembly, the needle assembly comprising:

      i) a needle housing defining a housing interior, said housing comprising the patient puncture tip extending from a first end of the housing and a non-patient puncture tip extending from a second end of the housing, and
      ii) a porous vent positioned within the housing interior and separating the housing interior into a first chamber and a second chamber, with the non-patient puncture tip and the patient puncture tip being in fluid communication with each other within the first chamber such that the sole communication path between the housing interior and the external environment is via the patient puncture tip, the porous vent including pores

the method **characterised in that** the pores are adapted for passage of blood therethrough from the first chamber into the second chamber;

b) establishing fluid communication between the non-patient puncture tip and an evacuated container such that blood contained within the first chamber is drawn out of the non-patient puncture tip and air is drawn out of the second chamber through the porous vent, thereby establishing a negative pressure within the second chamber relative to the external environment of the needle assembly such that blood flows through a cannula into the first chamber and contacts the porous vent; and

c) drawing blood through the pores of the porous vent toward the second chamber based upon the negative pressure established within the second chamber such that blood contained within a lumen of the patient puncture tip is displaced away from the patient puncture tip and toward the second chamber.

6. The method of claim 5, wherein the receiving step a) comprises receiving blood through the lumen of the patient puncture tip from a patient's bloodstream, and the drawing step c) displaces blood away from the patient puncture tip after removing the patient puncture tip from the patient's bloodstream.

7. The method of claim 5, including a further step after step b) and prior to step c) comprising releasing the fluid communication between the non-patient puncture tip and the pressure source.

8. The method of claim 7, wherein the pressure source comprises an evacuated collection container and wherein the step of releasing the fluid communication comprises removing the evacuated collection container from the non-patient puncture tip and sealing the non-patient puncture tip from the external environment.

9. The method of claim 8, wherein the needle assembly comprises a sealable sleeve extending about the non-patient puncture tip, said sealable sleeve being displaceable so as to permit the fluid communication between the evacuated collection container and the non-patient puncture tip in step b), and so as to reseal upon removal of the evacuated collection container from the non-patient puncture tip.

10. The method of claim 9, including a further step after said step of releasing the fluid communication between the non-patient puncture tip and the evacuated collection container, comprising establishing fluid communication between the non-patient puncture tip and a second evacuated collection container, such that blood is drawn through the lumen of the patient puncture tip and into the second evacuated collection container, followed by releasing the fluid communication between the non-patient puncture tip and the second evacuated collection container.

**Patentansprüche**

1. Nadelanordnung mit:

einem Gehäuse (412), das einen Gehäuseinnenraum definiert, wobei das Gehäuse eine Patientenpunktionsspitze (438) aufweist, die sich von einem ersten Ende (414) des Gehäuses erstreckt, und eine Nicht-Patientenpunktionsspitze (462) aufweist, die sich von einem zweiten Ende (416) des Gehäuses erstreckt, wobei die Nicht-Patientenpunktionsspitze und die Patientenpunktionsspitze miteinander in Fluidverbindung stehen, wobei der einzige Verbindungsweg zwischen dem Gehäuseinnenraum und einer äußeren Umgebung über die Patientenpunktionsspitze verläuft; und

einer porösen Lüftungseinrichtung (910), die in dem Gehäuseinnenraum positioniert ist, um den Gehäuseinnenraum in eine erste Kammer (426) und eine zweite Kammer (427) zu teilen, wobei die Patientenpunktionsspitze und die Nicht-Patientenpunktionsspitze in Fluidverbindung mit der ersten Kammer des Gehäuses stehen, wobei die poröse Lüftungseinrichtung Poren aufweist,

wobei die Anordnung **dadurch gekennzeichnet ist, dass** die Poren für den Durchtritt von Blut aus der ersten Kammer in die zweite Kammer ausgebildet sind,

wobei die erste Kammer, die zweite Kammer und die poröse Lüftungseinrichtung derart ausgestaltet sind, dass beim Einführen der Patientenpunktionsspitze in einen Patienten, Blut in die erste Kammer strömt, ohne die poröse Lüftungseinrichtung abzudichten, und dass beim Entfernen von mindestens einem Teil von Luft aus der zweiten Kammer Blut aus der ersten Kammer gesogen wird, wodurch ein negativer Druck in der zweiten Kammer relativ zu der äußeren Umgebung der Nadelanordnung hergestellt wird.

2. Nadelanordnung nach Anspruch 1, ferner mit einer einzelnen Kanüle, die sich durch das Gehäuse erstreckt, wobei die einzelne Kanüle ein sich dadurch erstreckendes Lumen, ein erstes Ende mit der Patientenpunktionsspitze, ein zweites Ende mit der Nicht-Patientenpunktionsspitze und eine Öffnung durch die Kanüle in das Lumen an einer Stelle zwischen dem ersten Ende und dem zweiten Ende aufweist, welche die Fluidverbindung zwischen dem Lumen der Kanüle und der ersten Kammer des Gehäuses bereitstellt.

3.  Nadelanordnung nach Anspruch 1, ferner mit einer ersten Kanüle, die sich von dem Gehäuse erstreckt und die Patientenpunktionsspitze aufweist, und einer zweiten Kanüle, die sich von dem Gehäuse erstreckt und die Nicht-Patientenpunktionsspitze aufweist, wobei die erste Kanüle und die zweite Kanüle in dem Gehäuseinnenraum im Wesentlichen axial ausgerichtet sind und durch einen Spalt voneinander getrennt sind, der in Fluidverbindung mit der ersten Kammer des Gehäuses steht.

4.  Nadelanordnung nach Anspruch 1, ferner mit einer abdichtbaren Hülse, die sich um die Nicht-Patientenpunktionsspitze herum erstreckt.

5.  Verfahren zum Verhindern des Austretens von Blut aus der Nadelanordnung mit folgenden Schritten:

    a) Aufnehmen von Blut durch eine Patientenpunktionsspitze und in eine erste Kammer der Nadelanordnung, wobei die Nadelanordnung aufweist:

    i) ein Nadelgehäuse, das einen Gehäuseinnenraum definiert, wobei das Gehäuse die Patientenpunktionsspitze aufweist, die sich von einem ersten Ende des Gehäuses erstreckt, und eine Nicht-Patientenpunktionsspitze aufweist, die sich von einem zweiten Ende des Gehäuses erstreckt, und
    ii) eine poröse Lüftungseinrichtung, die in dem Gehäuseinnenraum platziert ist und den Gehäuseinnenraum in eine erste Kammer und eine zweite Kammer teilt, wobei die Nicht-Patientenpunktionsspitze und die Patientenpunktionsspitze in der ersten Kammer derart in Fluidverbindung miteinander stehen, dass der einzige Verbindungsweg zwischen dem Gehäuseinnenraum und der äußeren Umgebung über die Patientenpunktionsspitze verläuft, wobei die poröse Lüftungseinrichtung Poren aufweist,

    wobei das Verfahren **dadurch gekennzeichnet ist, dass** die Poren für den Durchtritt von Blut aus der ersten Kammer in die zweite Kammer ausgebildet sind;
    b) Herstellen einer Fluidverbindung zwischen der Nicht-Patientenpunktionsspitze und einem evakuierten Behälter derart, dass das in der ersten Kammer enthaltene Blut aus der Nicht-Patientenpunktionsspitze gesogen wird und Luft durch die poröse Lüftungseinrichtung aus der zweiten Kammer gesogen wird, wodurch ein negativer Druck in der zweiten Kammer relativ zu der äußeren Umgebung der Nadelordnung derart hergestellt wird, dass Blut durch eine Kanüle in die erste Kammer fließt und in Kontakt mit der porösen Lüftungseinrichtung gelangt; und
c) Ziehen von Blut durch die Poren der porösen Lüftungseinrichtung in Richtung der zweiten Kammer basierend auf dem in er zweiten Kammer hergestellten negativen Druck derart, dass in einem Lumen der Patientenpunktionsspitze enthaltenes Blut von der Patientenpunktionsspitze weg und in Richtung der zweiten Kammer bewegt wird.

6.  Verfahren nach Anspruch 5, wobei der Aufnahmeschritt a) das Aufnehmen von Blut durch das Lumen der Patientenpunktionsspitze aus dem Blutstrom eines Patienten umfasst und der Entnahmeschritt c) Blut von der Patientenpunktionsspitze weg bewegt nach dem Entfernen der Patientenpunktionsspitze aus dem Blutkreislauf des Patienten.

7.  Verfahre nach Anspruch 5, ferner mit einem Schritt nach Schritt b) und vor Schritt c), der das Aufheben der Fluidverbindung zwischen der Nicht-Patientenpunktionsspitze und der Druckquelle umfasst.

8.  Verfahren nach Anspruch 7, wobei die Druckquelle einen evakuierten Sammelbehälter aufweist und wobei der Schritt des Aufhebens der Fluidverbindung das Entfernen des evakuierten Sammelbehälters von der Nicht-Patientenpunktionsspitze umfasst sowie das Abdichten der Nicht-Patientenpunktionsspitze gegenüber der äußeren Umgebung.

9.  Verfahren nach Anspruch 8, wobei die Nadelanordnung eine abdichtbare, sich um die Nicht-Patientenpunktionsspitze herum erstreckende Hülse aufweist, wobei die abdichtbare Hülse bewegbar ist, um die Fluidverbindung zwischen dem evakuierten Sammelbehälter und der Nicht-Patientenpunktionsspitze in Schritt b) zu ermöglichen, und um beim Entfernen des evakuierten Sammelbehälters von der Nicht-Patientenpunktionsspitze wieder abzudichten.

10. Verfahren nach Anspruch 9, mit einem weiteren Schritt nach dem Schritt des Aufhebens der Fluidverbindung zwischen der Nicht-Patientenpunktionsspitze und dem evakuierten Sammelbehälter, der das Herstellen einer Fluidverbindung zwischen der Nicht-Patientenpunktionsspitze und dem zweiten evakuierten Sammelbehälter umfasst , so dass Blut durch das Lumen der Patientenpunktionsspitze und in den zweiten evakuierten Sammelbehälter gesogen wird, wonach die Fluidverbindung zwischen der Nicht-Patientenpunktionsspitze und dem zweiten evakuierten Sammelbehälter aufgehoben wird.

## Revendications

1. Ensemble aiguille comprenant :

un boîtier (412) définissant un intérieur de boîtier, ledit boîtier comprenant une pointe de ponction côté patient (438) s'étendant à partir d'une première extrémité (414) du boîtier et une pointe de ponction côté opposé au patient (462) s'étendant à partir d'une deuxième extrémité (416) du boîtier, la pointe de ponction côté opposé au patient et la pointe de ponction côté patient étant en communication fluidique l'une avec l'autre, où le seul trajet de communication entre l'intérieur de boîtier et l'environnement extérieur est via la pointe de ponction côté patient ; et
un évent poreux (910) positionné à l'intérieur de boîtier pour séparer l'intérieur de boîtier en une première chambre (426) et une deuxième chambre (427), la pointe de ponction côté patient et la pointe de ponction côté opposé au patient étant toutes deux en communication fluidique avec la première chambre du boîtier, l'évent poreux comportant des pores
l'ensemble étant **caractérisé en ce que** les pores sont adaptés pour le passage du sang à travers ceux-ci de la première chambre à la deuxième chambre,
dans lequel la première chambre, la deuxième chambre et l'évent poreux sont configurés de sorte que, lors de l'insertion de la pointe de ponction côté patient dans le corps d'un patient, le sang s'écoule dans la première chambre sans sceller l'évent poreux et que lors du retrait d'au moins une partie de l'air de la chambre secondaire, le sang soit prélevé de ladite première chambre, établissant ainsi une pression négative dans ladite deuxième chambre par rapport à l'environnement extérieur de l'ensemble aiguille.

2. Ensemble aiguille de la revendication 1, comprenant en outre une canule unique s'étendant à travers le boîtier, ladite canule unique comportant une lumière s'étendant à travers celle-ci, une première extrémité comprenant la pointe de ponction côté patient, une deuxième extrémité comprenant la pointe de ponction côté opposé au patient et une ouverture à travers la canule dans la lumière à un emplacement entre la première extrémité et la deuxième extrémité assurant une communication fluidique entre la lumière de la canule et la première chambre du boîtier.

3. Ensemble aiguille de la revendication 1, comprenant en outre une première canule s'étendant à partir du boîtier et comprenant la pointe de ponction côté patient, et une deuxième canule s'étendant à partir du boîtier et comprenant la pointe de ponction côté opposé au patient, la première canule et la deuxième canule étant essentiellement alignées axialement dans ledit intérieur de boîtier et séparées l'une de l'autre par un intervalle en communication fluidique avec la première chambre du boîtier.

4. Ensemble aiguille de la revendication 1, comprenant en outre un manchon pouvant être scellé s'étendant autour de la pointe de ponction côté opposé au patient.

5. Procédé pour empêcher la fuite de sang d'un ensemble aiguille comprenant le fait :

a) de recevoir du sang à travers une pointe de ponction côté patient et dans une première chambre de l'ensemble aiguille, l'ensemble aiguille comprenant :

i) un boîtier d'aiguille définissant un intérieur de boîtier, ledit boîtier comprenant la pointe de ponction côté patient s'étendant à partir d'une première extrémité du boîtier et une pointe de ponction côté opposé au patient s'étendant à partir d'une deuxième extrémité du boîtier, et
ii) un évent poreux positionné à l'intérieur de boîtier et séparant l'intérieur de boîtier en une première chambre et une deuxième chambre, la pointe de ponction côté opposé au patient et la pointe de ponction côté patient étant en communication fluidique l'une avec l'autre dans la première chambre de sorte que le seul trajet de communication entre l'intérieur de boîtier et l'environnement extérieur soit via la pointe de ponction côté patient, l'évent poreux comportant des pores

le procédé étant **caractérisé en ce que** les pores sont adaptés pour le passage du sang à travers ceux-ci de la première chambre dans la deuxième chambre ;
b) d'établir une communication fluidique entre la pointe de ponction côté opposé au patient et un récipient sous vide de sorte que le sang contenu dans la première chambre soit extrait de la pointe de ponction côté opposé au patient et que l'air soit extrait de la deuxième chambre à travers l'évent poreux, établissant ainsi une pression négative dans la deuxième chambre par rapport à l'environnement externe de l'ensemble aiguille de sorte que le sang s'écoule à travers une canule dans la première chambre et entre en contact avec l'évent poreux ; et

c) de prélever le sang à travers les pores de l'évent poreux vers la deuxième chambre sur la base de la pression négative établie dans la deuxième chambre de sorte que le sang contenu dans une lumière de la pointe de ponction côté patient soit déplacé loin de la pointe de ponction côté patient et vers la deuxième chambre.

**6.** Procédé de la revendication 5, dans lequel l'étape de réception a) comprend la réception de sang à travers la lumière de la pointe de ponction côté patient de la circulation sanguine d'un patient, et l'étape de prélèvement c) permet de déplacer le sang loin de la pointe de ponction côté patient après retrait de la pointe de ponction côté patient de la circulation sanguine du patient.

**7.** Procédé de la revendication 5, comportant une étape supplémentaire après l'étape b) et avant l'étape c) comprenant la libération de la communication fluidique entre la pointe de ponction côté opposé au patient et la source de pression.

**8.** Procédé de la revendication 7, dans lequel la source de pression comprend un récipient de collecte sous vide et dans lequel l'étape de libération de la communication fluidique comprend le retrait du récipient de collecte sous vide de la pointe de ponction côté opposé au patient et le scellement de la pointe de ponction côté opposé au patient par rapport à l'environnement extérieur.

**9.** Procédé de la revendication 8, dans lequel l'ensemble aiguille comprend un manchon pouvant être scellé s'étendant autour de la pointe de ponction côté opposé au patient, ledit manchon pouvant être scellé pouvant se déplacer de manière à permettre la communication fluidique entre le récipient de collecte sous vide et la pointe de ponction côté opposé au patient dans l'étape b), et de manière à se resceller lors du retrait du récipient de collecte sous vide de la pointe de ponction côté opposé au patient.

**10.** Procédé de la revendication 9, comportant une étape supplémentaire après ladite étape de libération de la communication fluidique entre la pointe de ponction côté opposé au patient et le récipient de collecte sous vide, comprenant l'établissement d'une communication fluidique entre la pointe de ponction côté opposé au patient et un deuxième récipient de collecte sous vide, de sorte que le sang soit prélevé à travers la lumière de la pointe de ponction côté patient et dans le deuxième récipient de collecte sous vide, suivi de la libération de la communication fluidique entre la pointe de ponction côté opposé au patient et le deuxième récipient de collecte sous vide.

FIG. 1

FIG.2

FIG.3

EP 3 081 164 B1

FIG. 4.

FIG.5

FIG.6

FIG.7

EP 3 081 164 B1

410

430

436

461

438

**FIG.8**

436

410

461

430

**FIG.9**

FIG.10

EP 3 081 164 B1

FIG. 11A

FIG. 11B

24

FIG.12A

FIG.12B

EP 3 081 164 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5222502 A
- US 5303713 A

- WO 2006022716 A